# EUROPEAN PATENT SPECIFICATION

(11) **EP 1 409 033 B1**
(45) Date of publication and mention of the grant of the patent: **27.12.2006**
(21) Application number: 02752500.5
(22) Date of filing: 19.07.2002
(51) Int. Cl.: A61L 24/06

(54) **ADHESIVE INCLUDING MEDICAMENT**
MEDIKAMENT ENTHALTENDER KLEBSTOFF
ADHESIF RENFERMANT UN MEDICAMENT

(30) Priority: 19.07.2001 US 306572 P; 31.07.2001 US 308993 P; 07.11.2001 US 337662 P; 17.12.2001 US 341598 P; 07.01.2002 US 41688
(43) Date of publication of application: 21.04.2004
(73) Proprietor: LOMA LINDA UNIVERSITY MEDICAL CENTER, Loma Linda, CA 92350 (US)
(72) Inventor: ZHU, Yong-Hua, Redlands, CA 92373 (US); YANG, Chang, Zheng, Nanjing, Jiangsu 210008 (CN); SHEN, Qun-Dong, Loma Linda, CA 92350 (US); KIRSCH, Wolff, M., Redlands, CA 92373 (US); DICKSON, Cindy, Mentone, CA 92359 (US); GU, Min, Di, Nanjing, Jiangsu 210008 (CN)
(74) Representative: Otten, Hajo
(86) International application number: PCT/US2002/023222
(87) International publication number: WO 2003/008003

(56) References cited:
- WO-A-96/00760
- WO-A-96/10374
- WO-A-99/30685
- WO-A-99/42535
- US-A- 3 483 870
- US-A- 5 632 727
- US-A- 5 684 042

## Description

### Field of the Invention

The present invention provides medicament-containing cyanoacrylate adhesive formulations for sealing wounds.

### Background of the Invention

Wound closure technology continues to evolve with non-suture alternatives such as staples, surgical tapes, and most recently, tissue adhesives, which have rapidly gained recognition and acceptance as effective wound closure methods. Two different forms of tissue adhesives for wound closure have been extensively studied: cyanoacrylate tissue adhesives and fibrin sealants. Fibrin sealants have not gained acceptance because of the low tensile strength of the fibrin polymer, lengthy preparation time, and the risk of viral transmission. The cyanoacrylates are recognized as superior adhesives for skin wound closure and are undergoing continuous modification to improve the technology.

A common property of all of the cyanoacrylates is the ability to bond and polymerize in the presence of water and to form a bond between the two sides of a wound to hold it in position. When used for wound closure, the cyanoacrylate polymerizes in the presence of water molecules on the skin surface, forming a bridge and bond that keeps the tissue together for the purpose of wound healing. The polymerized material then progressively and slowly flakes off after holding the skin tissues in that position. The difficulties and hazards associated with the use of cyanoacrylates are well known. The cyanoacrylates are toxic and there may be adverse reactions because of hypersensitivity to cyanoacrylates themselves or formaldehyde, one of the starting materials used for preparing cyanoacrylate adhesives.

The first cyanoacrylates used as tissue adhesives included the short chain cyanoacrylates, commonly referred to as Super Glues^{™}, were associated with severe acute and chronic inflammatory reactions. Subsequently, longer chain cyanoacrylates, including butyl and octyl cyanoacrylates have gained acceptance. While butyl cyanoacrylates provide effective closure of simple superficial lacerations and incisions, they are toxic when introduced into vascular areas and exhibit low tensile strength and high brittleness.

Octyl cyanoacrylates have proved to be superior adhesives for wound closure, demonstrating greater tensile strength than the butyl cyanoacrylates, and are remarkably nontoxic when used for skin wound closure. Octyl cyanoacrylate has been approved by the FDA for use as a tissue adhesive. However, there are problems associated with its use, including a higher incidence of wound infection when compared to suturing as a wound closure method. Also, blood and body fluids trigger premature polymerization of the cyanoacrylate, resulting in an unsightly plasticized mass with very little skin bonding. It is also difficult to keep adhesive out of the wound. The polymerization reaction is exothermic, and the generated heat can result in patient discomfort. Octyl cyanoacrylates may have a low viscosity, causing them to run into undesirable areas or into the wound. For example, cyanoacrylates running into the eye can result in tarsorrhaphy (lid fusion) or corneal injury.

WO 99/42535 discloses cyanoacrylate adhesive compositions for sealing wounds that may comprise microencapsulated agents that reduce the formaldehyde concentration, i.e. "formaldehyde scavenger". The formaldehyde scavenger is effective to reduce active formaldehyde concentration levels reduced during in vivo biodegradation of the polymer. The adhesive may also comprise optionally a medicament.

WO 96/00760 discloses cyanoacrylate adhesives for sealing wounds comprising an effective amount of an pH-modifier effective to modifying the pH of an immediate in vivo environment of the adhesive at which the polymer of the adhesive biodegradates at a different rate than at physiologic pH. The pH-modifier can be microencapsulated to control biodegradation of the polymer.

WO 96/10374 discloses biomedical implant comprising a biomedical matrix, example given of cyanoacrylate, and a biodegradable porosifying agent. In additional to the matrix material and the porosifying agent the implant may further include therapeutic agents. The porosifying agent forms pores in the matrix so that the therapeutic agents can be released from the matrix.

### Summary of the Invention

There is a need in the art of wound closure for an adhesive that can close wounds with a reduced risk of infection, reduced bleeding, reduced pain, reduced tanning, and improved cosmetic appearance.

### Brief Description of the Drawings

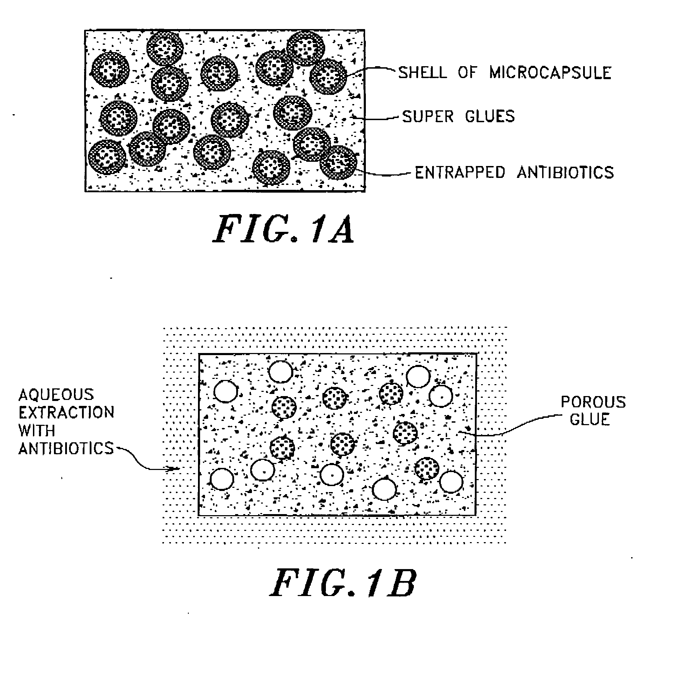

Figure la provides schematics illustrating release of an encapsulated medicament from a cyanoacrylate adhesive matrix. Figure 1a provides a schematic of a cross section of an adhesive matrix containing microcapsules. Figure 1b provides a schematic of the release of the microcapsules from the adhesive matrix.
Figure 2 provides IR spectra for Penicillin G, gelatin, and gelatin microcapsules of Penicillin G.
Figure 3 provides UV spectra for Penicillin G, gelatin, and gelatin microcapsules of Penicillin G.
Figure 4 provides UV spectra of a Sulfanilamide microcapsule extract at 10, 50 and 105 minutes.

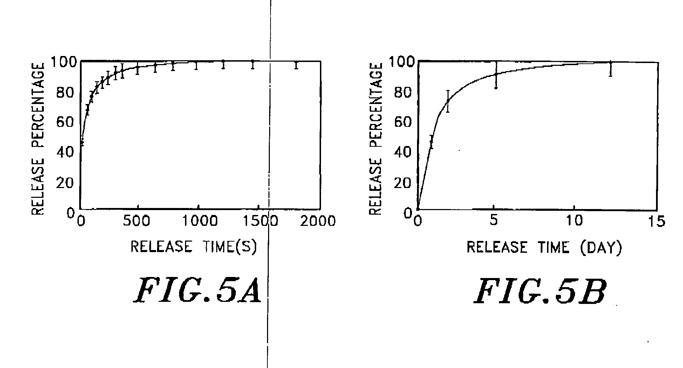

Figure 5a provides a release profile of gatifloxacin microcapsules prepared from an aqueous crosslinking solution (entrapment efficiency 2.3%, drug load 0.7%); and Figure 5b provides a release profile of gatifloxacin microcapsules prepared from a formaldehyde acetone crosslinking solution (entrapment efficiency 53%, drug load 6.7%).
Figure 6 provides UV spectra of extracts of encapsulated and unencapsulated Penicillin G in solidified cyanoacrylate film.
Figure 7 provides UV spectra of extracts of Sulfanilamide in smooth and rough solidified cyanoacrylate films.
Figure 8 provides the release curve (concentration versus time) of Sulfanilamidum from two portions of an adhesive film sample with sodium chloride as the defect forming agent.
Figure 9 provides the release curve (concentration versus time) of Sulfanilamidum from two portions of an adhesive film sample with polyethylene glycol.
Figure 10 provides the release curve (concentration versus time) of Gatifloxacin from an adhesive film sample with and without a polyethylene glycol defect forming agent.
Figures 11a and 11b are SEM images of the surface of a solidified adhesive containing 16.2 % PEG 600 before extraction with aqueous solution.
Figures 12a and 12b are SEM images of the surface of the adhesive of Figures 11a and 10b after extraction with aqueous solution.
Figure 13 shows the effect on the bacterial culture after exposure to Gatifloxacin on filter paper, and solidified adhesives including PEG only, microencapsulated Gatifloxacin only, and microencapsulated Gatifloxacin with PEG.
Figure 14 provides the release curves (release percentage versus time) of Gatifloxacin from adhesive films containing 0, 5.6, and 19 wt. % polyethylene glycol.
Figure 15 provides the release curves (release percentage versus time) of Gatifloxacin from adhesive films having thicknesses of 1 mm and 0.2 mm.
Figure 16 provides a schematic illustrating a separated package for antibiotic cyanoacrylate adhesive.
Figures 17a and 17b are optical microscope images of dexamethasone sodium phosphate-gelatin microcapsules.
Figure 18 provides release curves (release percentage versus time) for DST-gelatin microcapsules with different DST-gelatin feed ratios and crosslinking times.
Figure 19 provides HPLC chromatograms for DST solutions and an extractive solution of solidified adhesive film containing DST microcapsules.
Figure 20 provides the UV spectra of an extractive solution of Vitamin C microcapsules (VC-MC extract), extractive solution of Vitamin C microcapsule-containing adhesive film (VC-MC-SG extract), and aqueous solution of Vitamin C (VC solution).

### Detailed Description of the Preferred Embodiment

### Introduction

The following description and examples illustrate a preferred embodiment of the present invention in detail. Those of skill in the art will recognize that there are numerous variations and modifications of this invention that are encompassed by its scope. Accordingly, the description of a preferred embodiment should not be deemed to limit the scope of the present invention.

Minimally Invasive Surgery (MIS) surgery has lessened suffering of patients. Medical cyanoacrylate adhesives have been successfully used for effectively sealing the wounds acquired during such surgery, as well as for sealing other wounds such as lacerations. An embodiment described herein provides a medical cyanoacrylate adhesive that contains a medicament that can be released and delivered to the wound in a controlled fashion.

The medicament may be delivered to a wound that has been sealed with the disclosed adhesives. In a preferred embodiment, the medicament incorporated into the adhesive and delivered to the wound is encapsulated using known microencapsulation technologies. In other embodiments, the medicament is added directly to the adhesive. The adhesives of a preferred embodiment belong to the class of cyanoacrylate adhesives. In order to facilitate release of the medicament from the adhesive matrix, a defect or pore forming agent is formulated into the adhesive. Figure 1a provides a schematic of medicament-containing microcapsules incorporated within an adhesive matrix. The matrix may also include a defect or pore forming agent, typically a hydrophilic polymer or water soluble salt (Figure 1b). Upon contact with an aqueous solution (e.g., blood or tissue fluid), the defect or pore forming agent may be solubilized, leaving behind passageways into the interior of the adhesive matrix (Figure 1c). The microencapsulated medicament may then be released from the adhesive matrix through these defects or pores (Figure 1d).

The adhesives of preferred embodiments may possess various desirable properties, including, but not limited to, increased viscosity and improved curing rate. The use of the adhesives of preferred embodiments may permit various positive effects to be achieved, including, but not limited to, control of hemorrhaging, control of infection, control of pain, easier application of the adhesive, facilitated skin healing, and reduced tanning.

The term "entrapment efficiency," as used herein in conjunction with microencapsulated drugs, medicaments, or other substances, is a broad term and is used in its ordinary sense, including, without limitation, the weight of the entrapped drug in the microcapsule divided by the weight of the drug that follows a long-term release pattern.

The term "drug load," as used herein in conjunction with microencapsulated drugs, medicaments, or other substances, is a broad term and is used in its ordinary sense, including, without limitation, the weight of the entrapped drug, medicament, or other substance in the microcapsule divided by the weight of the microcapsule.

### Medicaments

Medicaments are penicillins (penicillin G sodium salt, amoxicillin, ampicillin, dicloxacillin, nafcillin, piperacillin, ticarcillin)

### Microencapsulated Medicaments

Certain medicaments, pharmaceutical compositions, therapeutic agents, and other substances desired to be incorporated into a cyanoacrylate medical adhesive may contain reactive groups that activate the polymerization of cyanoacrylic esters, resulting in premature curing of the adhesive. Other substances may be sensitive to the components of the cyanoacrylate adhesive and as a result may undergo adverse chemical reactions or become less active or nonactive. These effects may result in the inactivity of medicaments and failure of adhesives by solidification during storage. Microencapsulation is an effective technique to avoid undesired chemical interaction between medicaments and cyanoacrylates.

In a preferred embodiment, penicillin antibiotics are entrapped into hydrophilic gelatin microcapsules and mixed with cyanoacrylic ester adhesives. Other preferred shell materials include water-soluble alcohols and polyethylene oxides. The microcapsules' shells block undesired reactions by substantially preventing direct contact of the antibiotics and cyanoacrylates. Microencapsulation permits usage of a spectrum of antibiotics with appropriate sensitivity to different microorganisms. The microencapsulated antibiotics provide long-term controlled release of antibiotics from the solidified adhesives at a preselected concentration.

Microencapsulation techniques involve the coating of small solid particles, liquid droplets, or gas bubbles with a thin film of a material, the material providing a protective shell for the contents of the microcapsule. Microcapsules suitable for use in the preferred embodiments may be of any suitable size, typically from about 1 µm or less to about 1000 µm or more, preferably from about 2 µm to about 50, 60, 70, 80, 90, 100, 200, 300, 400, 500, 600, 700, 800, or 900 µm, and more preferably from about 3, 4, 5, 6, 7, 8, or 9 µm to about 10, 15, 20, 25, 30, 35, 40 or 45 µm. In certain embodiments, it may be preferred to use nanometer-sized microcapsules. Such microcapsules may range from about 10 nm or less up to less than about 1000 nm (1 µm), preferably from about 10, 15, 20, 25, 30, 35, 40, 45, 50, 60, 70, 80, or 90 nm up to about 100, 200, 300, 400, 500, 600, 700, 800, or 900 nm.

While in most embodiments a solid phase medicament or other substance is encapsulated, in certain embodiments it may be preferred to incorporate a liquid or gaseous substance. Liquid or gas containing microcapsules may be prepared using conventional methods well known in the art of microcapsule formation, and such microcapsules may be incorporated into the adhesives of the preferred embodiments.

### Microcapsule Components

The microcapsules of preferred embodiments contain a filling material. The filling material is typically one or more medicaments or other pharmaceutical formulations, optionally in combination with substances other than medicaments or pharmaceutical formulations. In certain embodiments, it may be preferred that the microcapsules contain one or more substances not including medicaments or pharmaceutical formulations. The filling material is encapsulated within the microcapsule by a shell material.

Typical shell materials include, but are not limited to, gum arabic, gelatin, ethylcellulose, polyurea, polyamide, aminoplasts, maltodextrins, and hydrogenated vegetable oil. While any suitable shell material may be used in the preferred embodiments, it is generally preferred to use an edible shell material approved for use in food or pharmaceutical applications. Such shell materials include, but are not limited to, gum arabic, gelatin, diethylcellulose, maltodextrins, and hydrogenated vegetable oils. Gelatin is particularly preferred because of its low cost, biocompatibility, and the ease with which gelatin shell microcapsules may be prepared. In certain embodiments, however, other shell materials may be preferred. The optimum shell material may depend upon the particle size and particle size distribution of the filling material, the shape of the filling material particles, compatibility with the filling material, stability of the filling material, and the rate of release of the filling material from the microcapsule.

### Microencapsulation Processes

A variety of encapsulation methods may be used to prepare the microcapsules of preferred embodiments. These methods include gas phase or vacuum processes wherein a coating is sprayed or otherwise deposited on the filler material particles so as to form a shell, or wherein a liquid is sprayed into a gas phase and is subsequently solidified to produce microcapsules. Suitable methods also include emulsion and dispersion methods wherein the microcapsules are formed in the liquid phase in a reactor.

### Spray Drying

Encapsulation by spray drying involves spraying a concentrated solution of shell material containing filler material particles or a dispersion of immiscible liquid filler material into a heated chamber where rapid desolvation occurs. Any suitable solvent system may be used, however, the method is most preferred for use with aqueous systems. Spray drying is commonly used to prepare microcapsules including shell materials including, for example, gelatin, hydrolyzed gelatin, gum arabic, modified starch, maltodextrins, sucrose, or sorbitol. When an aqueous solution of shell material is used, the filler material typically includes a hydrophobic liquid or water-immiscible oil. Dispersants and/or emulsifiers may be added to the concentrated solution of shell material. Relatively small microcapsules may be prepared by spray drying methods, e.g., from less than about 1 µm to greater than about 50 µm. The resulting particles may include individual particles as well as aggregates of individual particles. The amount of filler material that may be encapsulated using spray drying techniques is typically from less than about 20 wt. % of the microcapsule to more than 60 wt. % of the microcapsule. The process is preferred because of its low cost compared to other methods, and has wide utility in preparing edible microcapsules. The method may not be preferred for preparing heat sensitive materials.

In another variety of spray drying, chilled air rather than desolvation is used to solidify a molten mixture of shell material containing filler material in the form of particles or an immiscible liquid. Various fats, waxes, fatty alcohols, and fatty acids are typically used as shell materials in such an encapsulation method. The method is generally preferred for preparing microcapsules having water-insoluble shells.

### Fluidized-Bed Microencapsulation

Encapsulation using fluidized bed technology involves spraying a liquid shell material, generally in solution or melted form, onto solid particles suspended in a stream of gas, typically heated air, and the particles thus encapsulated are subsequently cooled. Shell materials commonly used include, but are not limited to, colloids, solvent-soluble polymers, and sugars. The shell material may be applied to the particles from the top of the reactor, or may be applied as a spray from the bottom of the reactor, e.g., as in the Wurster process. The particles are maintained in the reactor until a desired shell thickness is achieved. Fluidized bed microencapsulation is commonly used for preparing encapsulated water-soluble food ingredients and pharmaceutical compositions. The method is particularly suitable for coating irregularly shaped particles. Fluidized bed encapsulation is typically used to prepare microcapsules larger than about 100 µm, however smaller microcapsules may also be prepared.

### Complex Coacervation

A pair of oppositely charged polyelectrolytes capable of forming a liquid complex coacervate (namely, a mass of colloidal particles that are bound together by electrostatic attraction) can be used to form microcapsules by complex coacervation. A preferred polyanion is gelatin, which is capable of forming complexes with a variety of polyanions. Typical polyanions include gum arabic, polyphosphate, polyacrylic acid, and alginate. Complex coacervation is used primarily to encapsulate water-immiscible liquids or water-insoluble solids. The method is not suitable for use with water soluble substances, or substances sensitive to acidic conditions.

In the complex coacervation of gelatin with gum arabic, a water insoluble filler material is dispersed in a warm aqueous gelatin emulsion, and then gum arabic and water are added to this emulsion. The pH of the aqueous phase is adjusted to slightly acidic, thereby forming the complex coacervate which adsorbs on the surface of the filler material. The system is cooled, and a cross-linking agent, such as glutaraldehyde, is added. The microcapsules may optionally be treated with urea and formaldehyde at low pH so as to reduce the hydrophilicity of the shell, thereby facilitating drying without excessive aggregate formation. The resulting microcapsules may then be dried to form a powder.

### Polymer-Polymer Incompatibility

Microcapsules may be prepared using a solution containing two liquid polymers that are incompatible, but soluble in a common solvent. One of the polymers is preferentially absorbed by the filler material. When the filler material is dispersed in the solution, it is spontaneously coated by a thin film of the polymer that is preferentially absorbed. The microcapsules are obtained by either crosslinking the absorbed polymer or by adding a nonsolvent for the polymer to the solution. The liquids are then removed to obtain the microcapsules in the form of a dry powder.

Polymer-polymer incompatibility encapsulation can be carried out in aqueous or nonaqueous media. It is typically used for preparing microcapsules containing polar solids with limited water solubility. Suitable shell materials include ethylcellulose, polylactide, and lactide-glycolide copolymers. Polymer-polymer incompatibility encapsulation is often preferred for encapsulating oral and parenteral pharmaceutical compositions, especially those containing proteins or polypeptides, because biodegradable microcapsules may be easily prepared. Microcapsules prepared by polymer-polymer incompatibility encapsulation tend to be smaller than microcapsules prepared by other methods, and typically have diameters of 100 µm or less.

### Interfacial Polymerization

Microcapsules may be prepared by conducting polymerization reactions at interfaces in a liquid. In one such type of microencapsulation method, a dispersion of two immiscible liquids is prepared. The dispersed phase forms the filler material. Each phase contains a separate reactant, the reactants capable of undergoing a polymerization reaction to form a shell. The reactant in the dispersed phase and the reactant in a continuous phase react at the interface between the dispersed phase and the continuous phase to form a shell. The reactant in the continuous phase is typically conducted to the interface by a diffusion process. Once reaction is initiated, the shell eventually becomes a barrier to diffusion and thereby limits the rate of the interfacial polymerization reaction. This may affect the morphology and uniformity of thickness of the shell. Dispersants may be added to the continuous phase. The dispersed phase can include an aqueous or a nonaqueous solvent. The continuous phase is selected to be immiscible in the dispersed phase.

Typical polymerization reactants may include acid chlorides or isocyanates, which are capable of undergoing a polymerization reaction with amines or alcohols. The amine or alcohol is solubilized in the aqueous phase in a nonaqueous phase capable solubilizing the amine or alcohol. The acid chloride or isocyanate is then dissolved in the water- (or nonaqueous solvent-) immiscible phase. Similarly, solid particles containing reactants or having reactants coated on the surface may be dispersed in a liquid in which the solid particles are not substantially soluble. The reactants in or on the solid particles then react with reactants in the continuous phase to form a shell.

In another type of microencapsulation by interfacial polymerization, commonly referred to as *in situ* encapsulation, a filler material in the form of substantially insoluble particles or in the form of a water immiscible liquid is dispersed in an aqueous phase. The aqueous phase contains urea, melamine, water-soluble urea-formaldehyde condensate, or water-soluble urea-melamine condensate. To form a shell encapsulating the filler material, formaldehyde is added to the aqueous phase, which is heated and acidified. A condensation product then deposits on the surface of the dispersed core material as the polymerization reaction progresses. Unlike the interfacial polymerization reaction described above, the method may be suitable for use with sensitive filler materials since reactive agents do not have to be dissolved in the filler material. In a related in situ polymerization method, a water-immiscible liquid or solid containing a water-immiscible vinyl monomer and vinyl monomer initiator is dispersed in an aqueous phase. Polymerization is initiated by heating and a vinyl shell is produced at the interface with the aqueous phase.

### Gas Phase Polymerization

Microcapsules may be prepared by exposing filler material particles to a gas capable of undergoing polymerization on the surface of the particles. In one such method, the gas comprises p-xylene dimers that polymerize on the surface of the particle to form a poly(p-xylene) shell. Specialized coating equipment may be necessary for conducting such coating methods, making the method more expensive than certain liquid phase encapsulation methods. Also, the filler material to be encapsulated is preferably not sensitive to the reactants and reaction conditions.

### Solvent Evaporation

Microcapsules may be prepared by removing a volatile solvent from an emulsion of two immiscible liquids, e.g., an oil-in-water, oil-in-oil, or water-in-oil-in-water emulsion. The material that forms the shell is soluble in the volatile solvent. The filler material is dissolved, dispersed, or emulsified in the solution. Suitable solvents include methylene chloride and ethyl acetate. Solvent evaporation is a preferred method for encapsulating water soluble filler materials, for example, polypeptides. When such water-soluble components are to be encapsulated, a thickening agent is typically added to the aqueous phase, then the solution is cooled to gel the aqueous phase before the solvent is removed. Dispersing agents may also be added to the emulsion prior to solvent removal. Solvent is typically removed by evaporation at atmospheric or reduced pressure. Microcapsules less than 1 µm or over 1000 µm in diameter may be prepared using solvent evaporation methods.

### Centrifugal Force Encapsulation

Microencapsulation by centrifugal force typically utilizes a perforated cup containing an emulsion of shell and filler material. The cup is immersed in an oil bath and spun at a fixed rate, whereby droplets including the shell and filler material form in the oil outside the spinning cup. The droplets are gelled by cooling to yield oil-loaded particles that may be subsequently dried. The microcapsules thus produced are generally relatively large. In another variation of centrifugal force encapsulation referred to as rotational suspension separation, a mixture of filler material particles and either molten shell or a solution of shell material is fed onto a rotating disk. Coated particles are flung off the edge of the disk, where they are gelled or desolvated and collected.

### Submerged Nozzle Encapsulation

Microencapsulation by submerged nozzle generally involves spraying a liquid mixture of shell and filler material through a nozzle into a stream of carrier fluid. The resulting droplets are gelled and cooled. The microcapsules thus produced are generally relatively large.

### Desolvation

In desolvation or extractive drying, a dispersion filler material in a concentrated shell material solution or dispersion is atomized into a desolvation solvent, typically a water-miscible alcohol when an aqueous dispersion is used. Water-soluble shell materials are typically used, including maltodextrins, sugars, and gums. Preferred desolvation solvents include water-miscible alcohols such as 2-propanol or polyglycols. The resulting microcapsules do not have a distinct filler material phase. Microcapsules thus produced typically contain less than about 15 wt. % filler material, but in certain embodiments may contain more filler material.

### Liposomes

Liposomes are microparticles typically ranging in size from less than about 30 nm to greater than 1 mm. They consist of a bilayer of phospholipid encapsulating an aqueous space. The lipid molecules arrange themselves by exposing their polar head groups toward the aqueous phase, and the hydrophobic hydrocarbon groups adhere together in the bilayer forming close concentric lipid leaflets separating aqueous regions. Medicaments can either be encapsulated in the aqueous space or entrapped between the lipid bilayers. Where the medicament is encapsulated depends upon its physiochemical characteristics and the composition of the lipid. Liposomes may slowly release any contained medicament through enzymatic hydrolysis of the lipid.

### Miscellaneous Microencapsulation Processes

While the microencapsulation methods described above are generally preferred for preparing the microcapsules of preferred embodiments, other suitable microencapsulation methods may also be used, as are known to those of skill in the art. Moreover, in certain embodiments, it may be desired to incorporate an unencapsulated medicament or other substance directly into the cyanoacrylate adhesive. Alternatively, the medicament or other substance may be incorporated into a solid matrix of a carrier substance. In such embodiments, since the medicament or other substance and the cyanoacrylate will come into contact prior to curing of the adhesive, the medicament or other substance is preferably not substantially sensitive to the cyanoacrylate, and does not cause substantial premature curing of the adhesive prior to application. The microcapsules that are added to the adhesive may all be of the same type and contain the same medicaments or other substances, or may include a variety of types and/or encapsulated medicaments or other substances.

### Preferred Microencapsulated Medicaments

In preferred embodiments, penicillin antibiotics are encapsulated into hydrophilic gelatin microcapsules prior to incorporation in the cyanoacrylate adhesive so as to prevent undesired reactions between antibiotics and the cyanoacrylate.

### Cyanoacrylate Adhesives

The adhesives of the preferred embodiments include polymers of 2-cyanoacrylic esters, more commonly referred to as cyanoacrylates. Cyanoacrylates are hard glass resins that exhibit excellent adhesion to high-energy surfaces, such as skin, but do not form strong bonds with low energy materials, e.g., polyolefins, polytetrafluoroethylene (marketed under the name Teflon^{™}), and polyvinylchloride (commonly referred to as vinyl). Cyanoacrylate polymers are spontaneously formed when their liquid monomers are placed between two closely fitting surfaces. The excellent adhesive properties of cyanoacrylate polymers arises from the electron-withdrawing characteristics of the groups adjacent to the polymerizable double bond, which accounts for both the extremely high reactivity or cure rate, and their polar nature, which enables the polymers to adhere tenaciously to many diverse substrates.

### Cyanoacrylate Monomer Chemistry

Some of the more common cyanoacrylate monomers include, but are not limited to, the ethyl, methyl, isopropyl, allyl, n-butyl, isobutyl, methoxyethyl, ethoxyethyl, and octyl esters. Cyanoacrylate adhesives are manufactured and marketed worldwide by various companies including Loctite, a Henkel Company, of Rocky Hill, CT, SAFE-T-LOC International Corporation of Lombard, IL, SUR-LOK Corporation of Walworth, WI, and Elmers Products, of Columbus, OH, the manufacturer of the well-known Krazy Glue^{™}. The ability of cyanoacrylates to rapidly cure and bond to skin makes them particularly well suited for use as medical adhesives. Cyanoacrylate adhesives suitable for use as medical adhesives include octyl 2-cyanoacrylate marketed as Dermabond^{™} topical skin adhesive by Ethicon, Inc., a Johnson & Johnson Company, of Somerville, NJ, and butyl cyanoacrylate marketed as Vetbond^{™} by World Precision Instruments, Inc. of Sarasota, FL.

The 2-cyanoacrylic ester monomers are all thin, water-clear liquids with viscosities of 1-3 mPa. Only a few of the many esters that have been prepared and characterized are of any significant commercial interest. Methyl and ethyl cyanoacrylates are most commonly used for industrial adhesives. Cyanoacrylate adhesives for medical and veterinary use generally include the longer alkyl chain cyanoacrylates, including the butyl and octyl esters.

The base monomers are too thin for convenient use and therefore are generally formulated with stabilizers, thickeners, and property-modifying additives. The viscosities of such cyanoacrylate adhesives can range from that of the base monomer to thixotropic gels. The alkyl esters are characterized by sharp, lacrimatory, faintly sweet odors, while alkoxyalkyl esters are nearly odor free, but less effective adhesives.

### Bond Formation

Cyanoacrylate liquid monomers polymerize nearly instantaneously via an anionic mechanism when brought into contact with any weakly basic or alkali surface. Even the presence of a weakly basic substance such as adsorbed surface moisture is adequate to initiate the curing reaction. The curing reaction proceeds until all available monomer has reacted or until it is terminated by an acidic species. The time of fixture for cyanoacrylate occurs within several seconds on strongly catalytic surfaces such as skin to several minutes on noncatalytic surfaces. Surface accelerators or additives enhancing the curing rate may be used to decrease the time of fixture on noncatalytic surfaces. However, such accelerators and additives are generally not preferred for use in bonding skin due to the catalytic nature of the skin surface. The basic polymerization reaction includes the following initiation, propagation, and termination steps:

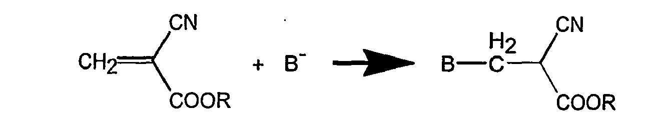

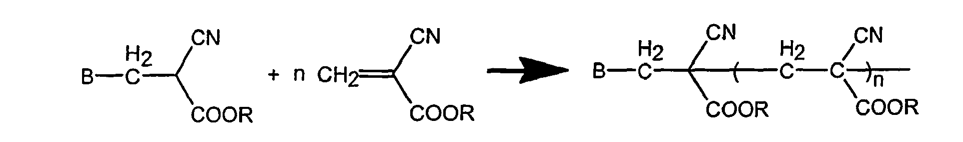

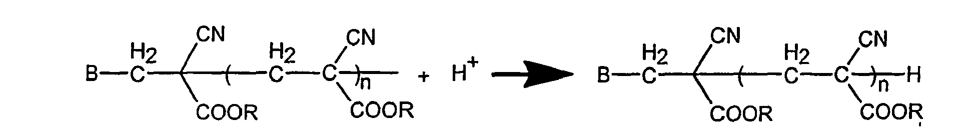

### Cyanoacrylate Adhesive Formulations

Cyanoacrylate adhesives are soluble in N-methylpyrrolidone, N,N-dimethylformamide, and nitromethane. Cured cyanoacrylates are hard, clear, and glassy thermoplastic resins with high tensile strengths, but tend to be brittle and have only low to moderate impact and peel strengths. Elastomeric materials may be dissolved in cyanoacrylate adhesive formulations to yield a cured adhesive of greater flexibility and toughness. The longer alkyl chain esters generally have longer cure rates, reduced tensile and tensile shear strength and hardness compared to the shorter alkyl chain esters. The longer alkyl chain esters also exhibit reduced glass-transition temperatures (T_{g}) and adhesive bond service temperature when compared to the shorter alkyl chain esters.

Although the alkyl cyanoacrylate esters are the most common cyanoacrylate adhesives, in certain embodiments it may be preferred to use a cyanoacrylate ester adhesive other than an alkyl ester. For example, allyl esters, which may cross-link by a free-radical mechanism through the allyl group, may be used in applications wherein increased thermal resistance is desirable. Alkoxyalkyl esters may be used in those applications where reduced odor is desirable and wherein a slightly reduced adhesive performance is acceptable.

Cyanoacrylate adhesives are prepared via the Knoevenagel condensation reaction, in which the corresponding alkyl cyanoacetate reacts with formaldehyde in the presence of a basic catalyst to form a low molecular weight polymer. The polymer slurry is acidified and the water is removed. The polymer is cracked and redistilled at a high temperature into a suitable stabilizer combination to prevent premature repolymerization. Strong protonic or Lewis acids are normally used in combination with small amounts of a free-radical stabilizer.

Adhesives formulated from the 2-cyanoacrylic esters typically contain stabilizers and thickeners, and may also contain tougheners, colorants, and other special property-enhancing additives. Both anionic and free radical stabilizers are required, since the monomer will polymerize via both mechanisms. Although the anionic polymerization mechanism depicted above is the predominant reaction, the monomer will undergo free radical polymerization under prolonged exposure to heat or light. To extend the usable shelf life of cyanoacrylate adhesive formulations, free-radical stabilizers such as quinones or hindered phenols are commonly added to the formulations. Anionic inhibitors such as nitric oxide may also be added. Such anionic inhibitors alter the viscosity and polymerization rate, thereby minimizing the risk of inadvertent spillage and facilitating application.

Both the liquid and cured cyanoacrylates support combustion, and highly exothermic polymerization can occur from direct addition of catalytic substances such as water, alcohols, and bases such as amines, ammonia, or caustics, or from contamination with surface activators.

### Cyanoacrylate Adhesives for Medical Uses

Cyanoacrylate adhesives will rapidly bond to skin because of the presence of moisture and protein in the skin. Octyl cyanoacrylates are the most widely used cyanoacrylate adhesive for tissue sealing. When bonding to tissue, octyl cyanoacrylates are four times stronger and less toxic than butyl cyanoacrylate. However, butyl cyanoacrylate is sometimes preferred for sealing deeper lacerations because it breaks down more easily and can be absorbed by the tissue more quickly than octyl cyanoacrylate.

The 2-cyanoacrylic esters have sharp, pungent odors and are lacrimators, even at very low concentrations. These esters can be irritating to the nose, throat, and eye at concentrations as low as 3 ppm. Good ventilation when using the adhesives is desirable and contact with the eye or other sensitive body parts is to be avoided when using cyanoacrylate adhesives for wound sealing. The cured 2-cyanoacrylic ester polymers are relatively nontoxic, making them suitable for medical use. While mild skin irritation may be observed, there is no evidence of sensitization or absorption of the cyanoacrylate adhesives through the skin.

### Defect or Pore Forming Additive for Adhesive

Cyanoacrylic esters form a dense structure after solidification which inhibits the penetration of medicaments contained within the adhesive into blood or tissues. Controlled release of medicaments from cyanoacrylate adhesives is typically achieved by one or more of the following routes: 1) biodegradation of cyanoacrylates in the presence of enzymes from blood or tissues around wound where the antiseptic glues are applied; 2) surface roughness or voids caused by non-uniform coating of adhesives to the wound; and 3) through artificially introduced defects in the adhesive matrix by mixing certain hydrophilic materials into the adhesive. When water comes in contact with the hydrophilic materials in the adhesive matrix, the materials are dissolved into the water and leave passages behind. These passages facilitate the controlled release of medicaments from microcapsules by allowing water to pass through the adhesive matrix.

In preferred embodiments, controlled release of medicaments from the adhesive matrix is primarily achieved through the use of artificially introduced defects or pores. Such defects may be induced using water-soluble salts, such as sodium chloride in powder form. However, in particularly preferred embodiments, polyethylene glycol (PEG) is added to the adhesive to form defects that provide passage to microencapsulated medicaments in the adhesive matrix, thereby increasing the releasing rate of the medicaments in the solidified adhesive film. PEG is generally preferred over water-soluble salts in that it yields a more homogeneous blend with cyanoacrylate adhesives than do water soluble salts such as sodium chloride.

Defects or passages for medicament release from a solidified adhesive film or matrix are preferably provided by adding PEG with an average molecular weight of 600 to the cyanoacrylate adhesive. While polyethylene glycol is the preferred defect-forming agent, defects may also be formed by adding any suitable hydrophilic material to cyanoacrylate adhesive. Suitable hydrophilic materials include, but are not limited to, water soluble or water miscible polymers, water soluble salts, water soluble small molecules, water soluble natural products, mixtures and combinations thereof, and the like.

Suitable water soluble polymers include, but are not limited to, polyethylene glycol (PEG), polyethylene glycol propionaldehyde, copolymers of ethylene glycol/propylene glycol, monomethoxy-polyethylene glycol, carboxymethylcellulose, dextran, polyvinyl alcohol (PVA), polyvinyl pyrrolidone, poly-1,3-dioxolane, poly-1,3,6-trioxane, ethylene/maleic anhydride copolymer, poly (β-amino acids) (including both homopolymers and random copolymers), poly(n-vinyl pyrrolidone)polyethylene glycol, polypropylene glycol homopolymers (PPG) and other polyakylene oxides, polypropylene oxide/ethylene oxide copolymers, polyoxyethylated polyols (POG) (e.g., glycerol) and other polyoxyethylated polyols, polyoxyethylated sorbitol, or polyoxyethylated glucose, colonic acids or other carbohydrate polymers, Ficoll or dextran and mixtures thereof. The water-soluble polymer used is preferably approved for clinical use.

Water-soluble polymers of any suitable molecular weight may be used. However, it is preferred that the molecular weight is selected such that the polymer chain is approximately the same length as that of the cyanoacrylate adhesive in which it is mixed. PEG with an average molecular weight of 600 provides satisfactory performance when mixed with Super Glue

The embodiments discussed above refer to cyanoacrylate adhesives. The methods of preferred embodiments, namely formation of pores or defects by solvation of a hydrophilic component in the adhesive matrix, may also be applied to adhesives of other chemistries. Preferably, such adhesives form matrices similar to those of cured cyanoacrylates, i.e., matrices that are substantially nonporous in the absence of additives, and substantially insoluble in water. Such adhesives may include, but are not limited to, epoxies, resins, and the like as are well known in the art. Such adhesives may be useful in applications other than wound sealing or other medical applications, i.e., applications wherein controlled release of a substance from the adhesive matrix under conditions of humidity or moisture is desirable.

It may also be desirable in certain embodiments to provide an adhesive that does not contain any medicament but which does have a faster degradation or disintegration rate than does the unadditized adhesive. For such applications, a defect-forming agent as described above may be added to the adhesive.

### Antidegradation Agents

Water-soluble acidic materials may slow down the polymerization and degradation rates of cyanoacrylates, thereby possibly reducing the toxicity of cyanoacrylate adhesives. Therefore, in certain embodiments it may be preferred to incorporate one or more physiologically acceptable organic or inorganic acids or salts of acids into the adhesive formulation. Suitable acids may be solid or in liquid form. Preferred are common basic, dibasic, or higher organic acids, including, but not limited to, malonic acid, mandelic acid, oxalic acid, lactic acid, lactobionic acid, fumaric acid, maleic acid, tartaric acid, citric acid, ascorbic acid, and acetic acid. Other suitable acids include physiologically acceptable dihydrogen phosphates and hydrogen sulfates, or physiologically acceptable salts of phosphoric acids (e.g., dihydrogen phosphates), sulfuric acids (e.g., dihydrosulfuric acid), hydrohalic acids (e.g., hydrochloric acids), and the like.

Suitable acid salts include, but are not limited to, physiologically compatible alkali or alkaline earth metal salts, especially sodium, potassium or calcium salts, as well as ammonium salts.

In addition to performing as antidegradation agents, the water soluble acidic materials may also act as pore forming agents. In certain embodiments wherein the acidic material functions as a pore forming agent, it may be preferred, to have an additional pore forming agent present, e.g., polyethylene glycol. Alternatively, in certain embodiments, the acidic material may be added to an adhesive formulation primarily because of its antidegradation activity in order to yield an adhesive of reduced toxicity. In such embodiments, the adhesive either may or may not contain one or more of a pore-forming agent, medicament, or any other additive as described above.

In order to provide antidegradation activity over an extended period of time, it may be preferred to add the acidic material to the adhesive in encapsulated form. Suitable encapsulation methods may include those described above for the preparation of microencapsulated medicaments.

In preferred embodiments, the water soluble acidic materials include Vitamin C (ascorbic acid), citric acid, and aspirin (salicylic acid). In particularly preferred embodiments, these acidic materials are provided as gelatine microcapsules.

The water soluble acidic material is preferably added to the cyanoacrylate at a concentration of from 0 wt. % to more than about 30 wt. %, more preferably from about 1, 2, 3, 4, 5, 6, 7, 8, or 9 wt. % to about 21, 22, 23, 24, 25, 26, 27, 28, or 29 wt. %, and most preferably from about 10 wt. % to about 11, 12, 13, 14, 15, 16, 17, 18, or 19 wt. %. The optimal concentration may depend upon the chemical composition, solubility, and acidity of the material, the chemical composition of the cyanoacrylate adhesive, whether the acid is present in encapsulated or unencapsulated form, and the rate of release of the acid if it is in encapsulated form, and level of acidity desired to be achieved. When the acidic substance is to be provided in encapsulated form, it is generally preferred that the microcapsules are from about 2 microns or less to about 100 microns or more in size, more preferably from about 5 microns to about 60, 70, 80, or about 90 microns, and most preferably from about 10, 15, 20, or 25 microns to about 35, 40, 45, or 50 microns. Preferred entrapment efficiencies are 20 wt. % or higher, more preferably 35 wt. % or higher, and most preferably from 50-80% or higher. The drug load is preferably from about 1 wt. % or less to 50 wt. % or more, and more preferably from about 5 wt. % to about 20 wt. %.

### Formulation of Adhesive Containing Microencapsulated Medicament

Microcapsules containing medicaments or other substances are prepared as described above. To ensure that premature curing of the adhesive does not occur upon addition of the microcapsules, it is desirable to ensure that the microcapsules are thoroughly dried. In preferred embodiments, the microcapsules are dried in the presence of a desiccant, and more preferably under a vacuum. After drying, the microcapsules are preferably maintained under a high purity inert atmosphere, e.g., dry nitrogen or argon, until they are added to the cyanoacrylate. Because basic compounds catalyze the polymerization of cyanoacrylate adhesives, it is desirable to control microcapsule and adhesive preparation so as to minimize the presence of such compounds.

The microcapsules and the defect formation agent may be added to the uncured cyanoacrylate adhesive in any convenient manner and in any convenient order. It is generally preferred to add the defect-forming agent to the uncured cyanoacrylate adhesive, then add the microcapsules to the resulting mixture. In order to form a homogenous mixture of adhesive, defect forming agent, and microcapsules, any suitable mixing method may be used, for example, mechanical stirring, shaking, or sonication. It is preferred that the mixing method not result in substantial damage of the microcapsules and the resulting premature release of medicaments or other substances contained therein. Preferably, the components are mixed and stored under an inert atmosphere or sealed in an airtight container prior to application.

The microcapsules are preferably added to the adhesive to provide a concentration of from less than about 5 wt. % to more than about 30 wt. %, more preferably from about 6, 7, 8, or 9 wt. % to about 21, 22, 23, 24, 25, 26, 27, 28, or 29 wt. %, and most preferably from about 10 wt. % to about 11, 12, 13, 14, 15, 16, 17, 18, or 19 wt. %. The optimal concentration may depend upon the concentration of filler material in the microcapsules, the type of medicament used, the desired release rate and dosage level of the medicament, the quantity and type of defect forming additive added to the cyanoacrylate, and the method of encapsulation used to prepare the medicament microcapsules. It is generally preferred that the active ingredient, whether incorporated into a microcapsule or added directly to the adhesive, be present in the adhesive at a concentration of from less than about 5 wt. % to more than about 30 wt. %, more preferably from about 6, 7, 8, or 9 wt. % to about 21, 22, 23, 24, 25, 26, 27, 28, or 29 wt. %, and most preferably from about 10 wt. % to about 11, 12, 13, 14, 15, 16, 17, 18, or 19 wt. %.

The water soluble defect forming material is preferably added to the cyanoacrylate at a concentration of from 0 wt. % to more than about 30 wt. %, more preferably from about 1, 2, 3, 4, 5, 6, 7, 8, or 9 wt. % to about 21, 22, 23, 24, 25, 26, 27, 28, or 29 wt. %, and most preferably from about 10 wt. % to about 11, 12, 13, 14, 15, 16, 17, 18, or 19 wt. %. The optimal concentration may depend upon the chemical composition and molecular weight of the water-soluble material, the chemical composition of the cyanoacrylate adhesive, the method of encapsulation used to prepare medicament microcapsules, and the rate of release and dosage level of the medicament.

In general, the more defect-forming agent added to the adhesive, the greater the release rate of medicament contained within the adhesive. Likewise, the smaller the molecular size or molecular weight of the water-soluble defect forming material, the greater the release rate.

In a preferred embodiment, the medicament is an antibiotic, the defect-forming additive is PEG, and the cyanoacrylate is octyl cyanoacrylate. The antibiotic is preferably encapsulated in a microcapsule having a gelatin shell and an average diameter of about 4 µm.

In certain embodiments, it may be desirable to add additional components to the adhesive. These additional components may include additives commonly used in cyanoacrylate adhesives, e.g., stabilizers and elastomers, as described above. Other materials may include fibers that improve the strength of the cured adhesive. Alternatively, after the adhesive has been applied to the wound but before it cures completely, a flexible woven or nonwoven fabric, or other similar sheet-like material, may be pressed on the surface of the adhesive. The fabric thus bonded to the adhesive improves the strength of the cured adhesive film.

The adhesive formulations of preferred embodiments may be used in any application wherein a conventional cyanoacrylate medical adhesive is used. The adhesives may be used to seal internal wounds (e.g., an artery incision), as well as external wounds (e.g., skin cuts, punctures, and lacerations). When the adhesive is to be used in sealing artery incisions, it is preferred that the adhesive has a burst strength exceeding 250 mmHg. However, in certain embodiments lower burst strengths may be suitable.

### Examples and comparative Examples

### Encapsulation of Antibiotics

Penicillin G Sodium Salt (hereinafter, "Penicillin G"), Sulfanilamidum Crystallinum Sterile (hereinafter "Sulfanilamide"), Cefalexin, and Gatifloxacin were selected as sample medicaments. Sulfanilamidum and Gatifloxacin were selected for testing in part because their ultraviolet-visible spectra are easily distinguished from the background spectrum observed for aqueous saline solution, and because their aqueous solutions are stable at ambient temperature.

Antiseptic microcapsules containing each of the antibiotics listed above were obtained by preparing an aqueous dispersion of the antibiotic and gelatin in liquid wax with vigorous stirring at 60°C. The dispersion was observed using visible microscopy to ensure the desired particle size was achieved. The dispersion was then cooled to 5°C while continuing to stir. The dispersion was then mixed with isopropanol and filtered to obtain the microcapsules. The microcapsules were treated with formalin solution, then the solution was stored in a refrigerator for about 24 hours. The solution was filtered to separate the microcapsules, which were thoroughly dried. The resulting antibiotic microcapsules were pale yellow and spherically shaped with a diameter of about 10 to 100 µm. Surfactants such as poly(vinyl alcohol) or Pluronic^{™} F68 may be used to stabilize the microcapsules and to provide a suitable particle size distribution. A narrow size distribution of microcapsules with a selected mean particle size can be obtained using conventional screening methods. The stability of the dispersion of microcapsules in the adhesive is largely dependent on the particle size.

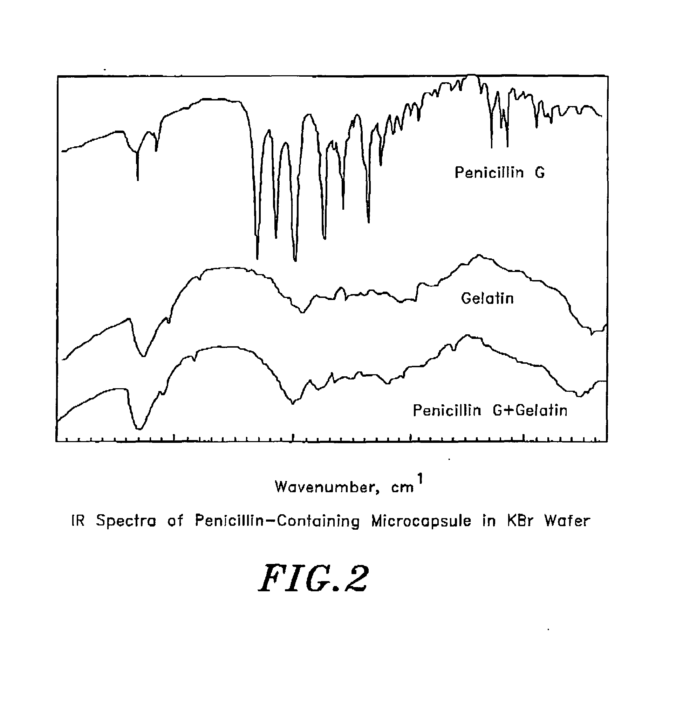

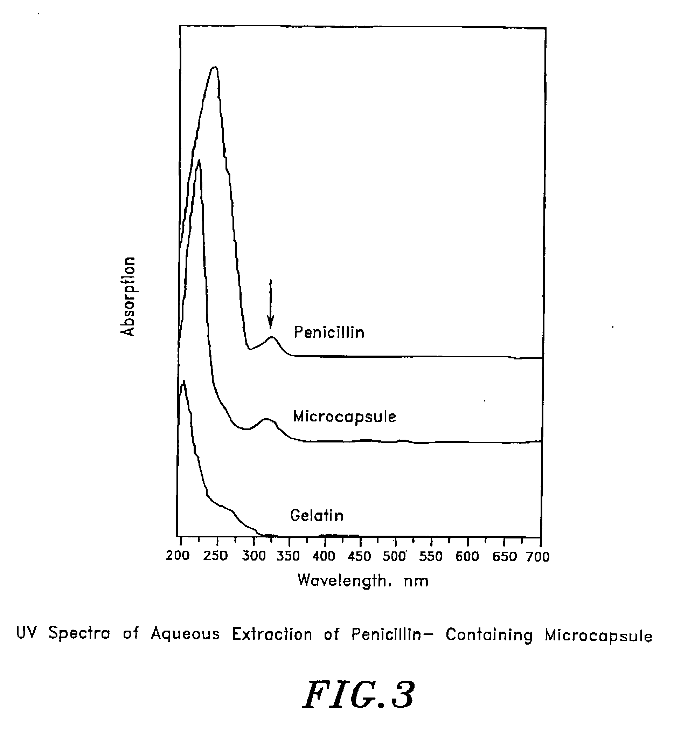

Antibiotics entrapped into gelatin microcapsules can be examined using infrared (IR) and ultraviolet (UV) spectroscopy. Potassium bromide wafers containing, respectively, Penicillin G, gelatin, and gelatin microcapsules of Penicillin G were examined using IR spectroscopy. As shown in Figure 2, there are no obvious peaks indicating the existence of Penicillin G in the spectrum for gelatin microcapsules for Penicillin G. However, UV spectra for aqueous extracts of, respectively, Penicillin G, gelatin, and gelatin microcapsules of Penicillin G yielded a notable absorption peak of Penicillin G for the extract of the gelatin microcapsules of Penicillin G (Figure 3). Because the penetrating ability of infrared light into the opaque microcapsules is rather weak, this suggests that Penicillin G may be mainly entrapped in the core rather than the shell, indicating successful microencapsulation.

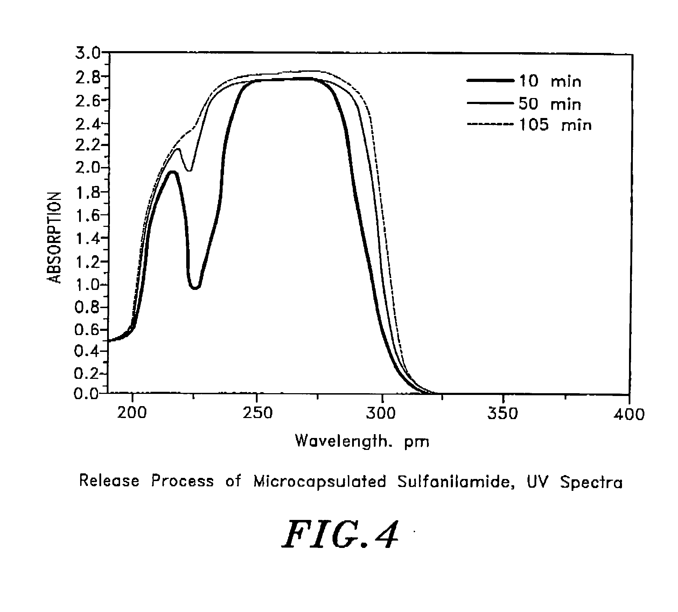

The release of antibiotic from the microcapsules was investigated by immersing either Penicillin G or Sulfanilamide microcapsules prepared as described above into a physiologic saline solution at body temperature. Penicillin G was observed to decompose during the release process. The aqueous extract of Sulfanilamide was stable at ambient temperature. Figure 4 provides UV spectra of the Sulfanilamide extract at 10, 50, and 105 minutes demonstrating the controlled release of Sulfanilamide from the microcapsules.

### Optimization of the Microcapsule Preparation Technique.

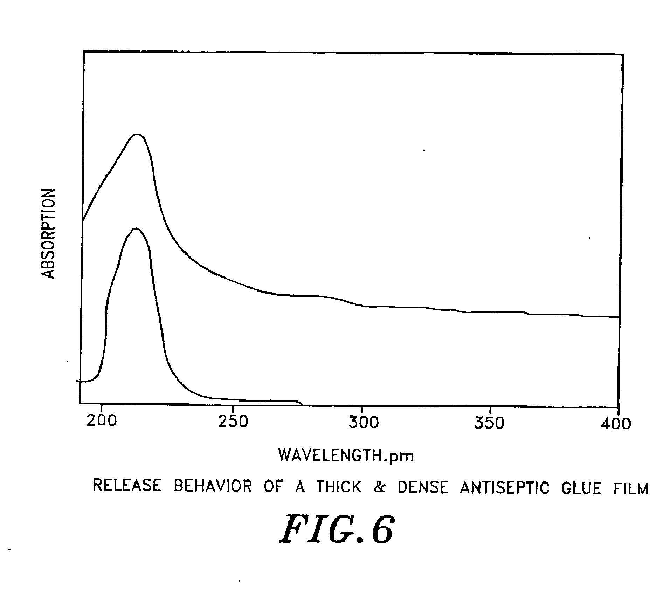

Microcapsules obtained by the initial process described in the previous section were observed to have a relatively low entrapment efficiency (<10%). Their release profile, provided in Figure 6a, did not follow a long-term release pattern. The release pattern indicates that about 80 percent of the total drug content was released in 2 minutes, suggesting that the drug was mainly adsorbed on the surface of the gelatin particles instead of being entrapped into gelatin matrices.

While not wishing to be limited to any particular mechanism, it is believed that the crosslinking step accounts, in part, for the encapsulation efficiency. A formaldehyde acetone solution was used as a crosslinking media because gatifloxacin displays a relatively weak solubility and gelatin does not swell in a formaldehyde acetone solution. Microcapsules with much higher entrapment efficiency (50-80%) were obtained using the modified process, and the microcapsules exhibited a long-term drug release profile as shown in Figure 6b.

Microcapsules with higher entrapment efficiencies may be prepared by adding 1 volume of an aqueous solution of gatifloxacin (typically about 1 to 10 wt. %), gelatin (typically about 20 wt. %), and Pluronic F-68 (available from Jinling Petroleum Chemical Co. Ltd. of China, typically present at about 1 wt. % as a stabilizer) into 8 volumes of liquid paraffin (available from Hangzhou Chemical Reagent Co. of China) with vigorous stirring at 60°C. The solution is stirred for about 15 min or until a whitish dispersion is formed. The dispersion is cooled to about 5°C and stirred for about 10 min to induce the full gelation of gelatin solution droplets. 30mL of a cold formaldehyde acetone solution (10 wt. %) is added to the system, which is stirred for another 20 min during which time crosslinking in the microcapsules occurs. The suspension is filtered and the filtered microcapsules are washed with cold acetone. The particles are vacuum dried at 40°C for 48 hours, yielding pale yellow spherical antibiotic microcapsules with size of about 10-50 microns.

The effects of crosslinking degree on the release profile of microcapsule were also studied but no significant impact was observed. A crosslinking time of 20 minutes was observed to yield satisfactory encapsulation efficiencies.

### Preparation of Unencapsulated Medicament Containing Adhesives

Adhesive formulations including unencapsulated antibiotics were investigated. The medicaments were vacuum dried for 6 hours at room temperature in the presence of phosphorous pentoxide to remove the residual water. Direct blending of the medicaments with cyanoacrylic ester was conducted in a drying chamber protected by a high-purity nitrogen atmosphere. Agglomeration was observed when Penicillin G was mixed with Super Glue, which may be due to initiation of the cyanoacrylate curing reaction by Penicillin. In contrast to Penicillin G, the shelf life of cyanoacrylate adhesives in the presence of Sulfanilamide was observed to be more than 24 hours. This suggests that the uncured cyanoacrylate is more sensitive to Penicillin G than Sulfanilamide.

### Preparation of Microcapsule Containing Adhesives

Adhesive formulations including encapsulated antibiotics were prepared. Microcapsules loaded with antibiotics were thoroughly dried under vacuum, then were evenly mixed with cyanoacrylate adhesives under oxygen-free and water-free conditions, then sealed. No agglomeration or solidification of cyanoacrylic ester was observed after 24 hours, suggesting that microencapsulation effectively suppresses the undesired chemical interaction between the medicaments and cyanoacrylic esters.

### Controlled Release of Antibiotics

Samples of adhesive containing either encapsulated Penicillin G or unencapsulated Penicillin G were prepared as described above. Solidification of the adhesives was carried out in moist air so as to provide an accelerated solidification rate. The solidified adhesives having a thickness of about 1 mm were cut into small pieces which were immersed in physiologic saline at room temperature. The aqueous extracts were examined using UV spectroscopy. As illustrated in Figure 6, no detectable release of Penicillin G (either encapsulated or unencapsulated) from the solidified adhesive film was observed. The lack of release may be attributed to the dense bulk of the cross-linked cyanoacrylic ester.

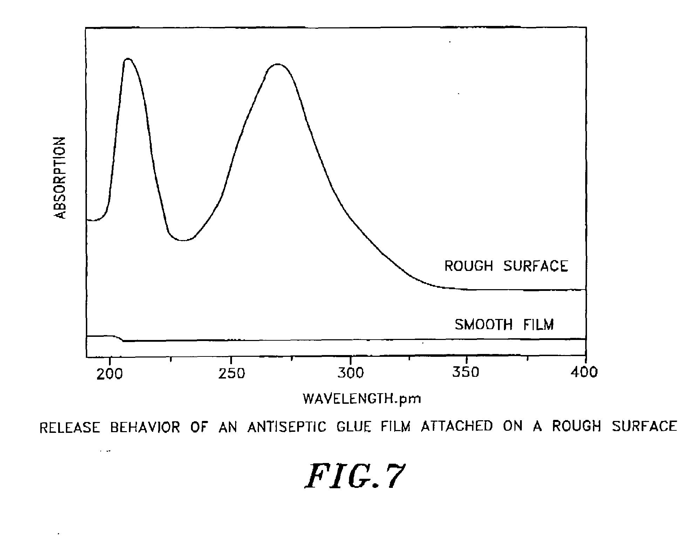

By reducing the thickness of solidified film, greater surface roughness and more voids are created, which may provide passages for the release of medicaments. Samples of adhesive containing Sulfanilamide were applied to filter paper infiltrated with ambient physiologic saline. UV spectroscopy of extracts of the solidified adhesives yielded the characteristic absorption of Sulfanilamide (Figure 7). It is believed that the rough and porous surface of the filter paper results in more defects in the solidified glue resulting after contact with the paper, which facilitates the releasing of the antibiotics.

### Artificially Formed Defects - Sodium Chloride Powder

Passages for drug release in dense solidified glue film were created through the use of pore forming or defect forming agents. Poly(ethylene glycol) with average molecular weight of 600 and sodium chloride powder were tested for their suitability as defect-forming agents selected.

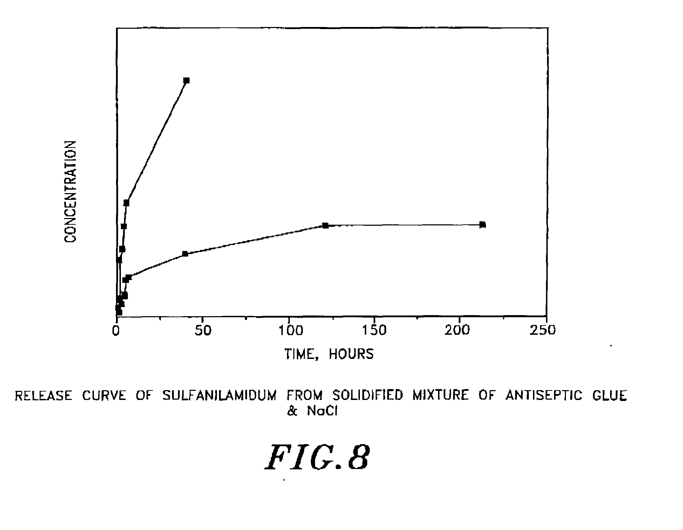

Aqueous extracts of the solidified adhesive prepared using sodium chloride displayed a UV absorption spectrum characteristic of sulfanilamide. However, a large variation in release rate was observed for different parts of a solidified adhesive film. Figure 8 provides release rate data for extracts from two different portions of the adhesive film. The data suggests that the blend is non-uniform due to the heterogeneous dispersion of the sodium chloride in the adhesive.

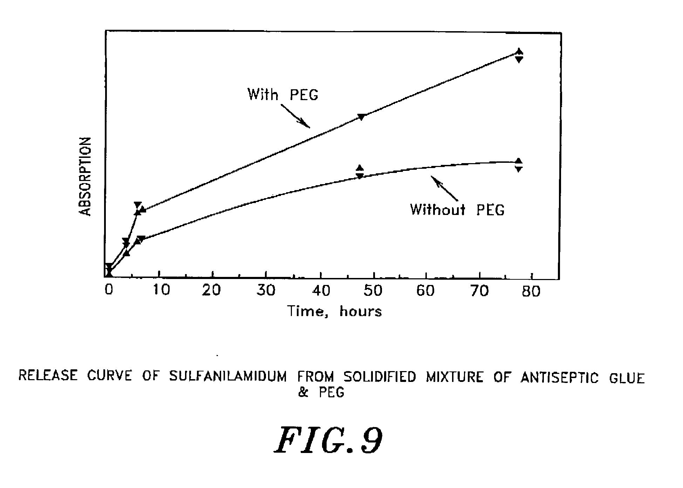

In contrast to the results observed for sodium chloride, an adhesive prepared using PEG demonstrated a more uniform release rate. Figure 9 provides release rate data for extracts from two different portions of the adhesive film.

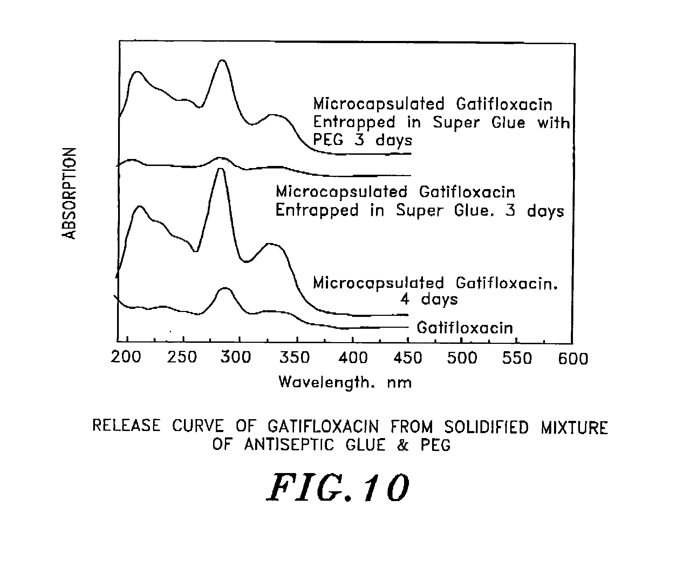

Adhesives were prepared using Gatifloxacin microcapsules both with and without PEG. Figure 10 shows the release characteristic of Gatifloxacin from the solidified adhesive film. As was observed in the experiments with Sulfanilamidum, incorporation of PEG also increased the release rate of Gatifloxacin in the solidified adhesive film.

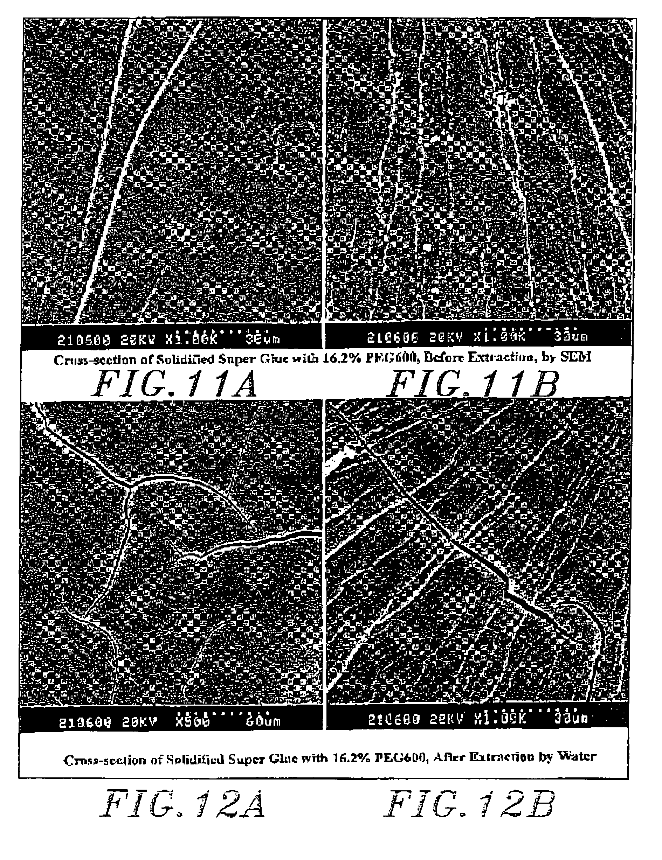

While not wishing to be limited to any particular mechanism, it is believed that when the solidified adhesive contacts an aqueous saline solution, PEG in the solid film is dissolved into the aqueous solution and leaves passage pores and defects behind. The microcapsules entrapped in the glue are thereby directly exposed to water in the channels formed by the defect generator, i.e., PEG. This process accelerates the diffusion of the antibiotic to the saline solution. Figures 11 a and 10b are SEM images of the surface of a solidified adhesive containing 16.2 % PEG 600 before extraction with aqueous solution. Figures 12a and 12b are SEM images of the surface of the same adhesive after extraction with aqueous solution. The solidified adhesive after extraction exhibits cracks and fissures not present before extraction.

### Microbiological Assay of Antibiotics Released from Adhesive

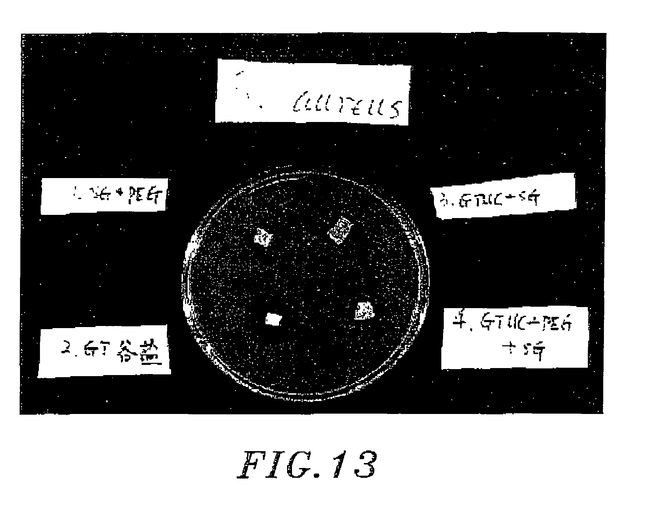

The antibiotic activity of different solidified adhesives was measured by placing small pieces of the solidified adhesive on a *S*. *aureus* bacterial culture. Figure 13 shows the effect on the bacterial culture after exposure to Gatifloxacin on filter paper (lower left-hand corner) and solidified adhesives including PEG only, microencapsulated Gatifloxacin only, and microencapsulated Gatifloxacin with PEG. (clockwise from the upper left hand corner of the image). The data demonstrate that superior releasability is observed for the antibiotic adhesive containing PEG.

### Release Behavior of Antibiotic Adhesives Containing Gatifloxacin Microcapsules

Polymerized cyanoacrylate forms a compact film that may inhibit the penetration of water into the adhesive matrix. Thus, the release of antibiotics from a well-formed polycyanoacrylate film may be difficult. As discussed above, introduction of PEG or defects into the adhesive matrix can greatly accelerate the release process.

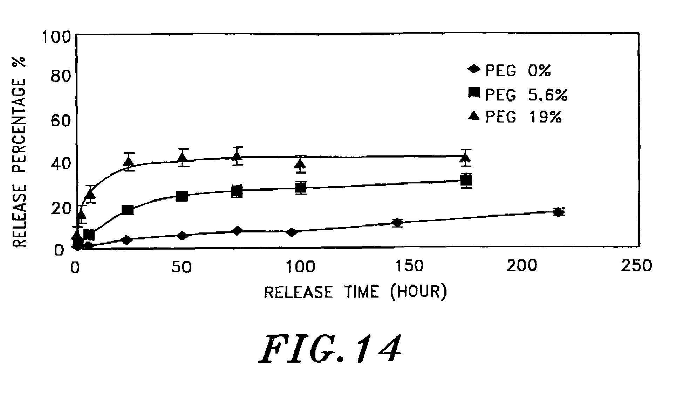

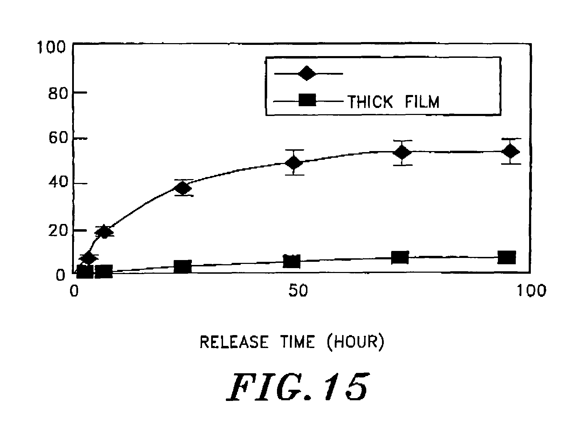

The release percentage for different polymerized cyanoacrylate films containing gatifloxacin microcapsules is illustrated in Figure 14 and Figure 15. Release percentage was calculated based on the total drug content of gatifloxacin microcapsules (6.7 wt. % drug load) entrapped in the solidified adhesive film. The microcapsule content (based on the total weight of the solidified adhesive) of the three films in Figure 14 (containing 0 wt. %, 5.6 wt. %, and 19 wt. % PEG, respectively) was 24 wt. %, 25 wt. %, and 26 wt. %, respectively. The microcapsule content of the films of Figure 15 was 25 wt. %. The thickness of the solidified adhesive films in Figure 14 and the thick film in Figure 15 was 1±0.1 mm. The thickness of the thin film in Figure 15 was approximately 0.2 mm.

The data illustrated in Figure 14 suggests that the presence of PEG in the adhesive matrix results in quicker release of antibiotic. The initial release rate rises significantly with the increase in PEG concentration. While not wishing to be limited to any particular mechanism, it is believed that the PEG within the solidified antibiotic adhesive is dissolved and leaves passages behind when the film contacts water. Thus, microcapsules entrapped in the dense film are exposed to water through those passages left by the dissolved PEG. This process may accelerate the diffusion of water into the solidified adhesive and speed up the drug release. It was noted that the adhesive containing 0 wt. % PEG also exhibited a weak release. It is believed that this is because of the presence of a small number of defects in the solidified adhesive film which led to the drug release. Experiment results also demonstrate that the drug release can be greatly accelerated when the thickness of the adhesive film is reduced, as shown in Figure 15. The data demonstrate that the drug release from the thin film having a thickness of about 0.2 mm was much quicker than that from the thick film having a thickness of about 1.0 mm.

It was noted, however, that the release percentages for the films of Figure 14 and Figure 15 is below 100%. It is believed that a certain amount of microcapsules were firmly encapsulated by polycyanoacrylate, and were not be able to get access to water until the outer polycyanoacrylate shell was degraded.

### Shelf Life of the Adhesive Containing Microcapsules

Direct mixing methyl cyanoacrylate (Super Glue^{™}) with dry gatifloxacin powder leads to solidification in about 3 hours at room temperature, and the color of cyanoacrylate turns to light green, indicating that some gatifloxacin has been dissolved in the Super Glue^{™}. However, a mixture of microencapsulated gatifloxacin and Super Glue^{™} exhibits superior stability. The shelf life of different cyanoacrylate adhesives containing 25 wt. % gatifloxacin microcapsules (6.7% drug load) is provided in Table 1.

**Table 1.**

| Cyanoacrylate | Methyl ester (Super Glue) | Ethyl ester (Adhesive 502 from Beijing Chemical and Engineering Company) | Butyl ester (Suncon Medical Adhesive from Beijing Suncon Medical Adhesive Co. Ltd.) |
|---|---|---|---|
| Shelf life (Room Temperature, about 25°C) | 5 days | 7 days | 10 days |
| Shelf life (4°C) | >20 days | >30 days | >40 days |

The data show that different cyanoacrylates have different reactivities, and thus different shelf lives. Typically, the higher alkyl ester cyanoacrylates have lower reactivity and longer shelf lives than the lower alkyl ester cyanoacrylates. The storage temperature also has a significant effect on the shelf life of adhesives. With reduced storage temperature, the shelf life was noticeably extended. Therefore, cold storage of antibiotic cyanoacrylate adhesives containing gatifloxacin microcapsules is preferred it is packed in single package.

In addition to chemical composition of the cyanoacrylate and storage temperature, the chemical composition or concentration of the pore forming agent, or the packaging process and container may also have a significant effect on the shelf life of adhesives containing microcapsules.

As illustrated in Figure 15, the addition of PEG can enhance the release of entrapped drug. However, PEG may have adverse effect on the stability of cyanoacrylate adhesive. Therefore, it is preferred to use a small amount of PEG (typically about 5 wt. % or less) if the adhesive is to be packed in single package. However, PEG may be substituted by other pore-forming materials in order to extend the shelf life of adhesives. Water-soluble acidic materials, such as Vitamin C, citric acid and aspirin, are preferred pore-forming agents because acidic substances may slow down the polymerization and degradation rates of cyanoacrylates, thereby possibly reducing the toxicity of cyanoacrylate adhesives.

Atematively, a separated package for antibiotic adhesives may be employed, thereby minimizing storage instability. A separated package is one wherein the cyanoacrylate adhesive and the pore forming agent and/or microencapsulated medicament are kept in different compartments and are mixed shortly before use. When such packaging is used, the content of PEG (or other pore-forming materials) may be raised to yield a satisfactory release rate and higher release percentage.

The presence of trace amount of basic substances, such as water and alcohol, may be sufficient to trigger the polymerization of cyanoacrylate adhesives. *(See* T. M. Brumit, "Cyanoacrylate adhesives - when should you use them?" *Adhesives Age,* February 1975, 17-22). It is therefore preferred that the amount of basic substances present be kept to a minimum in the mixture of cyanoacrylate and microcapsules. Thus, the packaging process may play a role in the resulting stability of antibiotic adhesives. Packaging processes which can effectively eliminate basic substances, including water, are expected to yield products with longer shelf lives. The container type may also be a factor in shelf life. For example, air-proof metal containers may provide the best storage stability, and polyethylene bottles or glass ampoules may also be satisfactory containers.

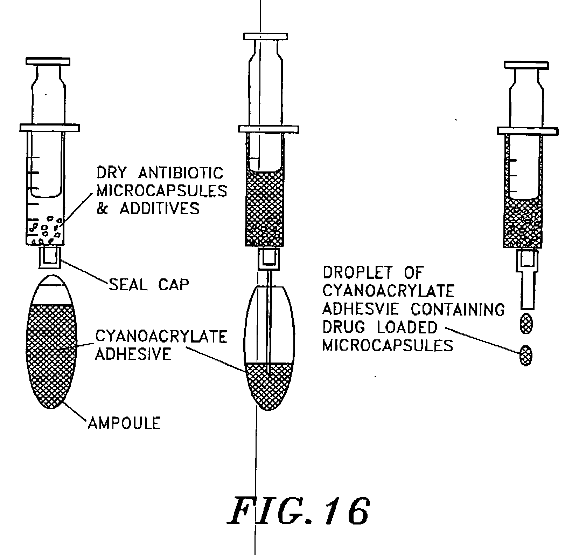

It is typically quite difficult to achieve satisfactory shelf life of cyanoacrylate-containing antibiotic microcapsules in a single package. Therefore, it is generally preferred to use a separated package form, as schematically depicted in Figure 16. The cyanoacrylate and microcapsules are separated in different containers which can easily be mixed shortly before use. Such a package form may provide satisfactory storage stability without the loss of operational convenience. Cyanoacrylate is typically stored in a sealed ampoule. Dry drug-loaded microcapsules and suitable additives such as PEG and Vitamin C are stored in a capped syringe. In order to prepare the adhesive for use, the seal cap on the syringe is removed and the ampoule that contains adhesive is opened. Cyanoacrylate is drawn into the syringe, which is shaken to thoroughly mix the adhesive and microcapsules. The mixture thus obtained may be extruded through a needle of suitable size. If the seal cap is put back onto the syringe, the mixture is able to maintain its fluidity for a period of time, typically for 4 or more hours. It is believed that a separated package will not only yield much longer shelf life but will also greatly reduce the production cost since the pretreatment (especially the drying process) of the microcapsules and containers may be simplified.

### Preparation of Dexamethasone Sodium Phosphate-Gelatin Microcapsules and Release of DSP from Solidified Adhesives Containing DSP Microcapsules

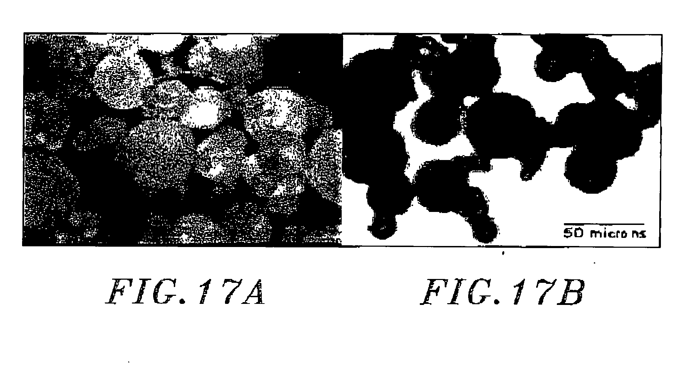

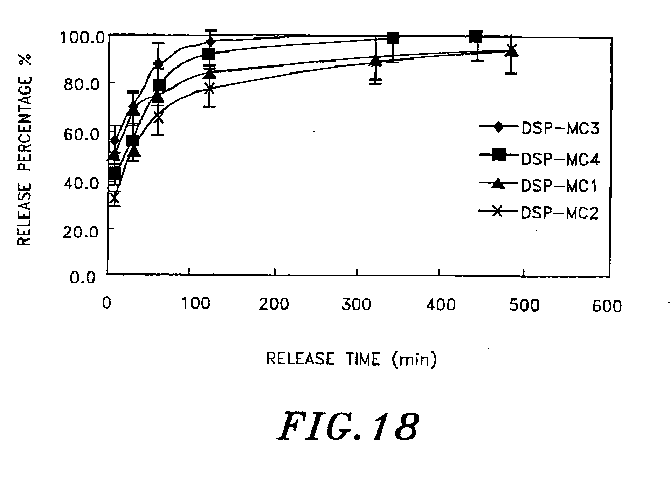

Dexamethasone Sodium Phosphate (DSP)-Gelatin microcapsules were prepared according to the optimized gelatin microcapsule method utilizing formaldehyde acetone crosslinking solution as described above. Figures 17a and 17b provide optical microscope images of the resulting DSP-Gelatin microcapsules. Preferably, the DSP concentration in the gelatin solution does not exceed 1 wt. %. If the DSP concentration in the gelatin solution is higher than 1%, the viscosity of the dispersion phase substantially increases, resulting in undesirably large (> 500 microns) microcapsules. *See* R. Arshady, "Microspheres and Microcapsules: A Survey of Manufacturing Techniques. Part 1: Suspension Cross-Linking," *Polym. Eng. and Sci.,* Dec. 1989, Vol. 29, No. 24, 1746-1758. At such low concentrations, the drug load of the resulting DSP microcapsule was low. However, the entrapment efficiency was satisfactory, as the data for four different batches of DSP microcapsules (DSP-MC1, DSP-MC2, DSP-MC3, and DSP-MC4) provided in Table 2 demonstrate. Moreover, the release profile of DSP microcapsules exhibited a long-term controlled release character, as illustrated in Figure 18.

**Table 2.**

| Microcapsule | DSP-MC1 | DSP-MC2 | DSP-MC3 | DSP-MC4 |
|---|---|---|---|---|
| Crosslinking time (min) | 210 | 30 | 210 | 30 |
| DSP/Gelatin feed ratio (w/w) | 0.028 | 0.028 | 0.050 | 0.050 |
| Drug load % | 1.86 | 2.23 | 3.46 | 3.54 |
| Entrapment efficiency % | 65.6 | 79.0 | 61.2 | 71.1 |

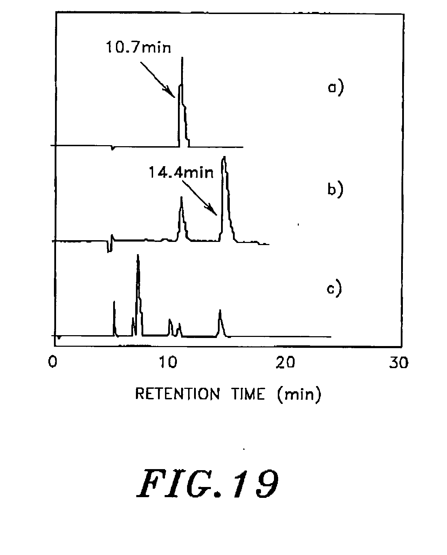

Because the UV spectra of DSP and the extractive aqueous solution, of Super Glue^{™} have overlapped absorptions at 240 nm, the release behavior of cyanoacrylate adhesives containing DSP microcapsules was studied by HPLC instead of UV spectroscopy. It was found that DSP microcapsules gradually decomposed in aqueous solution and its characteristic peak in the HPLC spectrum at a retention time of 10.7 min decreased and the peak at 14.4 min appeared and grew as the decomposition process progressed. Figure 19a shows the HPLC chromatogram of a DSP microcapsule solution prepared just before testing by HPLC, whereas Figure 19b shows the HPLC chromatogram of a DSP microcapsule solution prepared one month before testing by HPLC. The peak with a retention time of 14.4 min in Figure 19b is attributed to the decomposition product of DSP, and its area varies with storage time of the DSP aqueous solution.

The HPLC chromatogram of an extractive solution of solidified Super Glue^{™} film containing DSP microcapsules is shown in Figure 19c. The peak at 10.7 min is observable , indicating the release of DSP. The peak at 14.4 min is also observable, indicating that part of the DSP has decomposed during the storage of the extractive solution.

It is noted that if more effective dispersing methods, such as ultrasonication, vortex mixing and the like are used in the preparation of microcapsules, the particle size is expected to be reduced, and the drug load of DSP-gelatin microcapsules may be increased without an undesirable increase in size. A decrease in the microcapsule size may lead to better mechanical strength of solidified microcapsule-containing cyanoacrylate adhesive film.

### Reduction of Degradation Rates of 2-Cyanoacrylate Adhesives.

When 2-cyanoacrylates are used in medical applications, their biodegradability and the mechanism of degradation may play a role in their performance. The proposed degradation mechanism of ploy (2-cyanoacrylate) includes two possible pathways, illustrated below. The first mechanism is backbone degradation, which follows an inverse Knoevenagel reaction yielding formaldehyde and alkyl cyanoacetate. The other pathway is ester cleavage by side chain hydrolysis, resulting in poly (2-cyanoacylic acid) and alcohol.

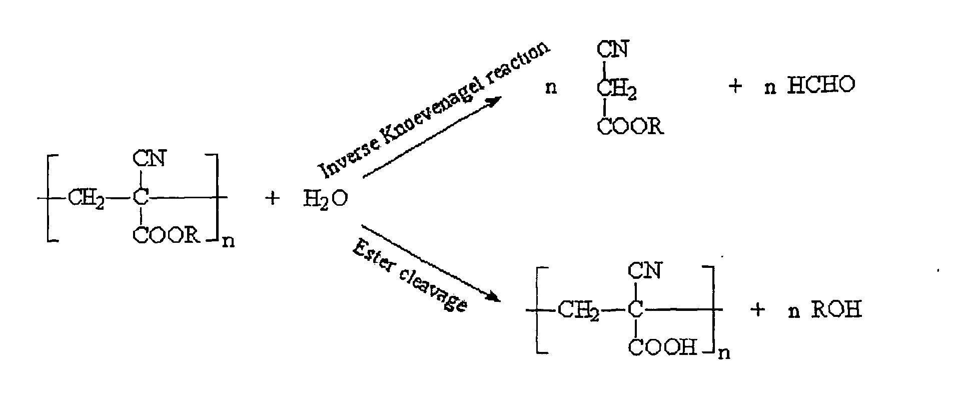

The second pathway appears to be the main mechanism. The degradation rate is dependent on the temperature, pH of the medium, enzyme content and length of the alkyl chains, and the toxicity is largely related to the degradation rates. If the degradation rates of solidified cyanoacrylate adhesive is decreased to such an extent that the products of degradation are instantly metabolized, then the adhesive may satisfy the requirements for medical use.

The degradation rate was observed to decrease with a decrease in temperature and pH value of the medium, and with an increase in the side ester chain length. Different kinds of enzymes and additives may accelerate or prohibit the degradation of poly(2-cyanoacrylate). For example, esterase may promote the degradation and superoxide dismutase, indomethacin and acetyl-salicylic acid may delay the degradation. Thus, butyl-and octyl-2-cyanoacrylate adhesives can be selected for medical use, and the cytotoxicity of the adhesive can be reduced by adjusting the pH value and/or enzyme content, and by addition of certain additives.

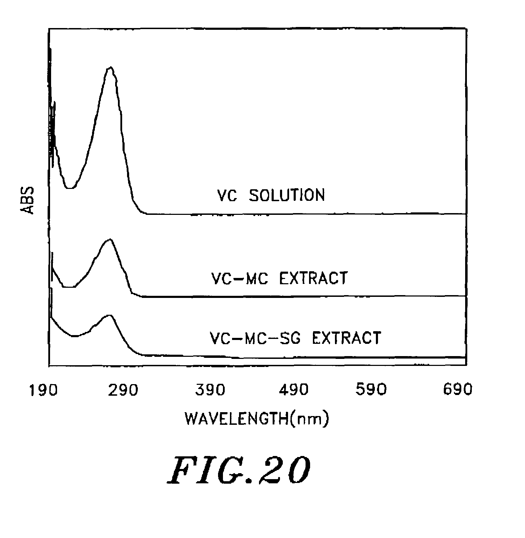

Because the degradation rate of poly(2-cyanoacrylate) is significantly reduced in a medium of pH<7, it is preferred to add certain microencapsulated physiologically-acceptable acidic materials to cyanoacrylate adhesives for a reduction of degradation rate and long term toxicity. Ascorbic acid (Vitamin C) gelatin microcapsules were prepared and the release behavior was qualitatively studied. The procedure for preparation of ascorbic acid-gelatin microcapsules is as described above except that a N₂ atmosphere was employed to avoid undesired oxidation of Vitamin C. The release of Vitamin C from solidified Vitamin C-gelatin microcapsule-containing adhesive film was observed by UV spectrometry. The spectrum, provided in Figure 20, indicates that the acidic environment of the solidified adhesive film may be maintained in this manner.

The experimental data demonstrate the feasibility of a medical cyanoacrylate adhesive with an antibiotic function. The preparation method of such antibiotic microcapsules plays a role in the performance of the adhesive. In order to ensure high entrapment efficiency, reasonable drug load and controllable microcapsule size, the preparation technique may be varied for different antibiotics. Gatifloxacin-gelatin microcapsules in the size range of 10-50 microns with 50-80 % entrapment efficiency and 5-20 % drug load prepared by the preparation technique utilizing formaldehyde acetone crosslinking solution provide generally satisfactory performance.

The experiment data also demonstrate that mixing an amount of PEG into a cyanoacrylate adhesive can increase the release rate of medicaments in the solidified film. The mechanical strength of solidified microcapsule-containing cyanoacrylate adhesive film may be noticeably reduced if the PEG content exceeds 30 wt. %, so it is preferred that the PEG be present at a concentration of 30 wt. % or less. The burst strength test of Super Glue^{™} containing microcapsules (20 wt. %) and PEG (20 wt. %) is satisfactory (burst strength > 350 mmHg). Typically, the mechanical strength of methyl cyanoacrylate (Super Glue^{™}) is higher than that of butyl or octyl cyanoacrylate.

When 2-cyanoacylates are used in medical applications, their biodegradability and the mechanism of degradation may be significant to the performance of the adhesive. The degradation rate is mainly dependent on the temperature, pH of the medium, enzyme content and length of the alkyl chains. The toxicity is largely related to the degradation rates. In general, if the degradation rate of the solidified cyanoacrylate adhesive decreased to such an extent that the products of degradation may be instantly metabolized, the adhesive may be suitable for use internally because of its low toxicity. Based on the fact that the degradation rate of poly(2-cyanoacrylate) is significantly reduced in a medium having a pH<7, a cyanoacrylate adhesive containing ascorbic acid-gelatin microcapsules may be preferred. The addition of acidic substances (Vitamin C, citric acid, and the like) into cyanoacrylate adhesives may retard their polymerization and degradation, and thus lower their toxicity such that butyl- or octyl cyanoacrylate adhesives may be able to meet the requirements of internal medical use. The addition of acidic substances to ethyl cyanoacrylate adhesive (Krazy Glue^{™}) may also make it suitable for medical purposes such as skin wound bonding, which may decrease the cost of medical adhesives because cost of ethyl cyanoacrylate is much lower than that of butyl- or octyl cyanoacrylate.

The shelf life of cyanoacrylate adhesive mixed with antibiotic microcapsules in a single package may be limited, and the addition of PEG may have adverse effects on the storage stability of cyanoacrylate. A separated package for antibiotic adhesives may provide a low cost and effective solution to providing satisfactory shelf life without losing operational convenience. And in this manner, more flexibility may be achieved since different combinations of cyanoacrylate and drug-loaded microcapsules and/or additives can be easily employed to meet different practical demands.

The above description discloses several methods and materials of the present invention. This invention is susceptible to modifications in the methods and materials, as well as alterations in the fabrication methods and equipment. Such modifications will become apparent to those skilled in the art from a consideration of this disclosure or practice of the invention disclosed herein. Consequently, it is not intended that this invention be limited to the specific embodiments disclosed herein, but that it cover all modifications and alternatives coming within the true scope of the invention as embodied in the attached claims.

## Claims

1. Stable liquid adhesive for sealing a wound, the adhesive comprising a cyanoacrylate, a therapeutic agent comprising a penicillin antibiotic encapsulated in a microcapsule, and a defect forming agent, wherein the defect forming agent is capable of being removed from a cured cyanoacrylate matrix by solvation in an aqueous solution whereby a plurality of defects in the matrix are formed permitting release of the therapeutic agent from the matrix at a controlled rate.

2. The adhesive of claim 1, wherein the cyanoacrylate comprises butyl cyanoacrylate.

3. The adhesive of claim 1, wherein the cyanoacrylate comprises octyl cyanoacrylate.

4. The adhesive of claim 1, wherein the defect forming agent comprises a hydrophilic polymer.

5. The adhesive of claim 4, wherein the hydrophilic polymer comprises polyethylene glycol.

6. The adhesive of claim 5, wherein the polyethylene glycol has an average molecular weight of about 600.

7. The adhesive of claim 1, further comprising a water-soluble acidic antidegradation agent.

8. The adhesive of claim 7, wherein the water-soluble acidic antidegradation agent comprises Vitamin C.

9. The adhesive of claim 1, wherein the microcapsule comprises a gelatin microcapsule.

10. The adhesive of claim 1, wherein the penicillin antibiotic comprises Penicillin G.

## Patentansprüche

1. Stabiler flüssiger Klebstoff zum Verschluss einer Wunde, wobei der Klebstoff ein Cyanoacrylat, einen therapeutischen Stoff mit einem in eine Mikrokapsel verkapselten Penicillin-Antibiotikum, sowie einen Defekt-bildenden Stoff aufweist, wobei der Defekt-bildende Stoff durch Lösen in einer wässrigen Lösung aus einer ausgehärteten Cyanoacrylatmatrix entfernt werden kann, wodurch eine Vielzahl von Defekten in der Matrix gebildet werden, wodurch die Freisetzung des therapeutischen Stoffes aus der Matrix mit einer kontrollierten Rate ermöglicht wird.

2. Klebstoff nach Anspruch 1, wobei das Cyanoacrylat Butylcyanoacrylat aufweist.

3. Klebstoff nach Anspruch 1, wobei das Cyanoacrylat Octylcyanoacrylat aufweist.

4. Klebstoff nach Anspruch 1, wobei der Defekt-bildende Stoff ein hydrophiles Polymer aufweist.

5. Klebstoff nach Anspruch 4, wobei das hydrophile Polymer Polyethylenglycol aufweist.

6. Klebstoff nach Anspruch 5, wobei das Polyethylenglycol ein durchschnittliches Molekulargewicht von ungefähr 600 besitzt.

7. Klebstoff nach Anspruch 1, der ferner einen wasserlöslichen sauren Anti-Abbaustoff aufweist.

8. Klebstoff nach Anspruch 7, wobei der wasserlösliche saure Anti-Abbaustoff Vitamin C aufweist.

9. Klebstoff nach Anspruch 1, wobei die Mikrokapsel eine Gelatine-Mikrokapsel aufweist.

10. Klebstoff nach Anspruch 1, wobei das Penicillin-Antibiotikum Penicillin G aufweist.

## Revendications

1. Adhésif liquide stable pour fermer hermétiquement une blessure, ledit adhésif comprenant un cyanoacrylate, un agent thérapeutique comprenant un antibiotique à base de pénicilline encapsulé dans une microcapsule, et un agent porogène, dans lequel l'agent porogène est capable d'être retiré d'une matrice de cyanoacrylate durcie, par solvatation dans une solution aqueuse, moyennant quoi on forme une pluralité de pores dans la matrice, permettant le relargage (ou la diffusion) de l'agent thérapeutique de la matrice à une vitesse contrôlée.

2. Adhésif selon la revendication 1, dans lequel le cyanoacrylate comprend du butyl cyanoacrylate.

3. Adhésif selon la revendication 1, dans lequel le cyanoacrylate comprend de l'octyl cyanoacrylate.

4. Adhésif selon la revendication 1, dans lequel l'agent porogène comprend un polymère hydrophobe.

5. Adhésif selon la revendication 4, dans lequel le polymère hydrophobe comprend du polyéthylène glycol.

6. Adhésif selon la revendication 5, dans lequel le polyéthylène glycol a un poids moléculaire moyen d'environ 600.

7. Adhésif selon la revendication 1, comprenant un agent anti-dégradation acide soluble dans l'eau.

8. Adhésif selon la revendication 7, dans lequel l'agent anti-dégradation acide soluble comprend de la vitamine C.

9. Adhésif selon la revendication 1, dans lequel la microcapsule comprend une microcapsule en gélatine.

10. Adhésif selon la revendication 1, dans lequel l'antibiotique à base de pénicilline comprend de la pénicilline G.
